Europäisches Patentamt

(19) European Patent Office

Office européen des brevets

(11) Publication number: **0 259 183**
**A2**

## EUROPEAN PATENT APPLICATION

(21) Application number: **87307844.8**

(51) Int. Cl.⁴: **C 12 Q 1/18**

(22) Date of filing: **04.09.87**

(30) Priority: 05.09.86 US 903709    05.09.86 US 903713
05.09.86 US 903724    05.09.86 US 903717
05.09.86 US 903712

(43) Date of publication of application:
**09.03.88 Bulletin 88/10**

(84) Designated Contracting States:
**BE CH DE ES FR GB IT LI LU NL SE**

(71) Applicant: **LIFETRAC**
**Suite 920 18300 Von Karman Avenue**
**Irvine California 92715 (US)**

(72) Inventor: **Baker, Fraser Lee**
**7675 Phoenix Apt. 2223**
**Houston Texas 77030 (US)**

(74) Representative: **Goldin, Douglas Michael et al**
**J.A. KEMP & CO. 14, South Square Gray's Inn**
**London WC1R 5EU (GB)**

The application is published incomplete as filed (Article 93 (2) EPC). The point in the description or the claim(s) at which the omission obviously occurs has been left blank.

(54) **Process for controlling the accuracy and precision of sensitivity assays.**

(57) A method of determining the relative clinical potency of a test agent, such as a chemotherapeutic drug or radiation, on cells from an individual. A plurality of assay cell samples from the individual are treated with a different doses of the agent, and the growth of the cell samples measured. An "inhibitory value" is then calculated, which is the value of one of the dose and dose response, corresponding to a selected value of the other one. For drug testing, the inhibitory value is preferably the value of the dose which produces a dose response selected at a 90% kill. The inhibitory value can then be compared with inhibitory values for cells from a plurality of other individuals to ascertain the relative clinical potency of the test agent. A number of techniques are provided which reduce errors associated with such assays so that meaningful comparisons can be made.

EP 0 259 183 A2

## Description

Processes for Controlling the Accuracy and Precision of Sensitivity Assays.

FIELD OF THE INVENTION:

The invention relates to methods for measuring the effective toxicity of various agents for cells by conducting tissue culture tests of their sensitivity (sensitivity tests) to various chemical and physical agents such as, but not exclusive to, chemotherapeutic drugs and radiation.

TECHNOLOGY REVIEW:

It is important to be able to accurately quantitate the response of tumor cells to therapeutic drugs on an individual-level, eg. so that the most effective therapy may be selected for a particular patient, and on a research-level, eg. for testing new drugs or drug combinations. Conducting cell growth in tissue culture is a standard practice in cell biology, biochemistry, and medical research including cancer research. In cancer research, one purpose for growing cells in this manner is to test the toxicity of various drugs for tumor cells as opposed to normal cells. Toxicity can be manifest in these cultures as either slower growth rate of the tumor cells, or as outright destruction of the cells by the cancer therapy agent.

One in vitro technique provides for growth of cells in a 'semi-solid' agar suspension system. Salmon et.al., U.S. Patent No. 4,411,990, discloses a method for growing malignant cells in a two layer gel system. On the bottom layer of the gel contains a tissue culture "feeder" layer composed of nutrient mediumand a gelling agent to solidify the medium. On the top is placed the tumor cells to be tested in a liquid nutrient medium suspension containing a gellable agent. When this medium is gelled it forms a second layer on top of the first, in which the cells can grown as discrete 'colonies' or 'clones' with each colony or clone arising from a single parent cell. Toxicity of a drug in this system is seen as a decrease in the number of colonies or clones which can be manually counted at the end of a period of growth. This type of assay, and variations of it, have been termed 'clonogenic' assays. Clonogenic assays have proven to have several disadvantages. First, large numbers of tumor cells are usually required because most cells do not grow in the assay or do not grow well. Second, it is technically difficult to form an even suspension of tumor cells where the individual cells are not in contact with one another as 'clumps' or 'aggregates', and if a uniform suspension of cells can not be prepared for clonogenic assays then there is the possibility that error will be introduced into the assay. Sara Rockwell describes these types of problems in EFFECT OF CLUMPS AND CLUSTERS ON SURVIVAL MEASUREMENTS WITH CLONOGENIC ASSAYS, Cancer Research, 45: 1601-1607, 1985. Third, in practice counting colonies is a highly subjective technique because colonies which are grow close to one another may mature into overlapping colonies which cannot be distinguished apart. Fourth, the practice of counting colonies is time consuming, labor intensive, and not readily automated. Because of technical problems only 30-40% of tumor samples surgically removed from patients will grow in clonogenic assays. There is also considerable uncontrolable technical error in clonogenic assays causing the results to vary significantly. As evidence of the commercial nonviability of this approach a clonogenic assay marketed by Roche under trademark 'Chemchek, tm' was introduced in 1984 and withdrawn from the market in 1985 because of poor sales and scientific criticism.

In traditional monolayer tissue culture techniques the cells are grown directly on the culture surface, eg. plastic or glass, because the proliferation of many cells can only occur after they have adhered to the culture surface. This is also true for tumor cells contained in biopsy materials obtained from many cancer patients. The growth characteristics of these types of adherent cells has been termed 'anchorage-dependent'. Solving the technical problem of 'anchorage-dependent' growth for tumor cells has been a major scientific goal for the past > 20 years.

With very few possible exceptions, all cells are intimately surrounded in tissues and organs by material termed 'extracellular matrix'. Extracellular matrix adheres cells to one another, provides barrier, and may also serve a function in controling cell growth and differentiation during embryonic development. Factors have been isolated from extracellular matrix which promote the adhesion of tumor cells to surfaces, and some of these factors also promote the growth of 'anchorage-dependent' tumor cells in tissue culture. Such factors have for example been described by:

ALPIN, et.al., "Complex Carbohydrates of the Extracellular Matrix Structures, Interactions, and Biological Roles", Bioch. Biophys. Acta, Vol, 694, pp. 375-418 (1983), discloses, at page 398, second column, that fibronectin is known to promote both attachment and spreading of specified cell types, including tumor cells, on plastic and glass.

LIOTTA, et.al., "Biochemical Interactions of Tumor Cells with the Basement Membrane", Ann. Rev. Biochem., Vol, 55, pp. 1037-1057, 1986, discloses the importance of laminin, type IV collagen, heparan sulfate proteoglycan, and fibronectin in tumor cell growth.

Growth factors capable of promoting tumor cell growth in tissue culture have been described. They have been isolated from a variety of biological sources, eg. serum, plasma, tissue culture medium in which tumor cells have been grown, and platelets. Some growth factors are known to promote the growth of certain types of tumor cells in tissues culture. For example such are described by:

MACIAG et.al., "Hormonal Requirements of Baby Hamster Kidney Cells in Culture", Cell Biology International Reports, January, 1980 discloses, in the Abstract, the proliferation of baby hamster kidney cells in synthetic medium supplemented with insulin, transferrin, fibroblast growth factor, and epidermal growth factor on cell culture dishes coated with fibronectin.

At present, the best composition for growing tumor cells containing the most effective combination of adhesion factors and the best combination of growth factors is not known. However, many recent advances permit routine growth of tumor cells on surfaces coated with adhesion factors. Commercial examples are extracellular matric material (ECM) marketed by IBT, Jerusalem, Israel; plates coated with cell adhesive matrix (CAM) marketed by LifeTrac, Irvine, California; and, collagen for coating plates (Vitrogen) marketed by Collagen Corporation, Palo Alto, California. These methods enable one skilled in the art to routinely grow tumor cells on specially-coated surfaces with good outgrowth of tumor cells from surgical tumor materials.

These recent advances in growing tumor cells have served to focus attention on the significant technical impediments hindering the routine testing of tumor cells in sensitivity assays for chemotherapeutic drugs or radiation. Namely, these impediments include technical problems with cell aggregation and clumping (discussed above ); problems in quantitating cell growth; problems in accurately controling for differences in tumor cell growth rates and for variations in the quality of the inoculum (which may result in cell overgrowth or undergrowth) and their influence on the final test results; and, problems in calculating accurately the toxicity of different agents for tumor cells. The following several paragraphs illustrate and expand on the importance of these technical problems.

Cell aggregation, (i.e., clumping), and uneven distribution resulting from redistribution of the tumor cells in the various tissue culture wells constituting the assay chambers, both have the effect that colonies of tumor cells may arise from a cluster containing 2 or more 'parent' colony forming cells. In radiation sensitivity testing, this means that these cell colonies will require twice as much radiation to kill as if the colony had arisen from a single colony forming unit. When plotting such 'flawed' data the results show artificial 'plateaus' in the radiation survival curve which either skew the overall results or make an interpretation of the results difficult because it appears that the tumor may be composed of cells with two or more different type of radiation sensitivities.

An additional technical problem involves the fluidity of the culture medium resulting in uneven distribution of cells on the bottom of the assay chambers normally used for sensitivity testing. Because of the high fluidity of fluid liquid medium, more tumor cells may be distributed around the edges of the tissue culture well than in the center due to the motion during handling and to vibrations in the environment. Again, this is a variable which is not presently controled in most assays, and it can have a significant impact on the results because colonies at the edge of well are difficult to accurately count.

Problems in subjectively quantitating cell growth, such as by colony counting, can result in large errors in the number of colonies counted. Since the number of tumor cells available for testing is usually limited, assays are most commonly conducted with only two replicates of the tumor sample for each treatment dose of drug or radiation. The duplicate results are averaged to obtain the final assay value. Therefore, the cumulative effect of averaging the individual errors can be quite substantial and can result in some cases in a tumor being considered to be resistant to a particular drug while it is in fact sensitive, which would be shown if more quantitative techniques are used. Therefore, any assay system attempting to rank the relative sensitivity/resistance of different tumors to chemotherapeutic drugs, radiation, or other agents requires accurate and precise data.

Differences in tumor cell growth efficiencies are common even between tumors of the same histological type. This makes it difficult for one skilled in the art to be able to predict how many cells to use to inoculate the tissue culture plates. If too many cells are used, the tumor cells will overgrow the assay wells before the end of the assay and many cells may either die as a result, or they may grow on top of one another, making it impossible to quantitate the amount of growth. In addition, it is important that the toxic effects of cancer therapeutic agents be tested when the tumors are in the actively growing logarithmic-phase of growth. This is particularly important with agents which may slow the growth of cells, but not directly kill cells. In the latter case, if the cell cultures are inoculated with too many cells it may not be possible at the end of the culture period to observe the growth slowing effect of these types of therapeutic agents, and the possible benefit to the patient may be completely missed. If too few cells are used, it will also be impossible to quantitate the growth.

In addition to the above problems, tissue samples available from surgical biopsies of human tumors vary considerably in their composition. One problem arising from this is that nondisrupted clumps or tissue fragments in the sample which may be inappropriately interpreted as reflecting cellular growth when quantitating the assay. This introduces a random error which in the extreme case can lead to uninterpretable results.

Given the aforementioned technical problems, it becomes a very significant consideration how one skilled in the art chooses to calculate and express the final data so that appropriate quantitative controls are included in the calculations, and so that tumor samples obtained from different patients can be quantitatively compared for their relative degree of responsiveness (or nonresponsiveness) to therapeutic agents.

Previously it has not been possible to establish the relative clinical potency of a test agent in cells from an individual (that is, relative to other individuals). This was due to the large errors previously associated with quantitating cell growth as already described. Such errors made meaningful comparisons of clinical potency of a test agent between individuals, virtually impossible.

In summary, it has become very important to find ways to technically improve the accuracy of in vitro assays for measuring the sensitivity of tumor cells to drugs and physical agents (eg. radiation).

Therefore, it would be highly desirable to have a method for routinely preventing the aggregation of tumor cells so that they can be accurately distributed into tissue culture plates. It would also be highly desirable to have a method for routinely counting the growth of cell colonies which is well suited to automation and computer analysis. It would also be highly desirable to have a method for controling for the individual variation in the growth rates of cellular samples obtained from different individuals. It would also be advantageous to be able to quantitate the extent to which non-dividing cells or tissue fragments in the test samples contribute to the 'background noise' to the values obtained in the assays, so that they can be subtracted out of the final results. It would also be desirable to provide a method for accurately handling the data obtained in sensitivity assays to calculate the toxicity of different drugs and physical agents.

SUMMARY OF THE INVENTION

The invention provides uniform methods for measuring the effective toxicity of various agents for cells by conducting tissue culture tests of their sensitivity to various chemical and physical agents such as, but not exclusive to, cancer chemotherapeutic drugs and radiation. Other applications may relate to testing the toxicity of agents for normal cells, eg. environmental safety testing of the effects of chemical toxins or occupational health and safety testing of agents encountered in the workplace. The invention provides: a) a method for preventing cell aggregation in a liquid suspension and of evenly distributing cells; b) a method for controling for variations in cell proliferation efficiency; c) an optical density method for measuring the toxicity of agents for cells in sensitivity assays; d) a background subtraction technique for use in the assay as a control; and, e) methods to calculate survival curves to establish treatment efficacy. These various methods can be practiced in combination or independently. In combination they comprise a controlled and uniform system for testing the sensitivity of tumor cells or normal cells to various test agents, with a high degree of accuracy. Thus, the relative clinical potency of a test agent can be established with a good degree of reliability.

The invention provides a method for insuring uniform suspensions of tumor cells which eliminates problems with the fluidity of liquid culture medium. The method comprises incorporating an effective amount of viscosity modifier into the medium in which the cells are suspended. The viscosity modifier is not used to form a gel or cross-linked matrix, if this happens it is undesirable.

The viscosity modifier is preferably, but not exclusively, methylcellulose, pectin, a plant gum, or a chemical viscous material such as, but not limited to, sucrose. where the viscosity modifier is methylcellulose it is added in the amount of 0.6%. According to the invention the cell suspension contains about 2,000-500,000 cells/ml in a suitable liquid such as, but not exclusive to, Dulbecco's Modified Eagles Minimal Essential Medium (D-MEM).

The invention is further directed to a cell suspension for incorporation into the assay chamber comprised of:
    a) a cell sample;
    b) a suspension liquid; and,
    c) viscosity modifier in an amount sufficient to substantially eliminate peripheral distribution of cells due to capillarity and to increase the uniformity of the cell distribution as they are deposited onto the cell culture surface.

The invention is further directed to a method of assuring uniform cell distribution in the cell suspension which comprises incorporating an effective amount of viscosity modifier into the cell suspension without forming a substantially cross-linked gel matrix. Such a method may involve subsequently aliquoting portions of the dispersed suspension into the cell culture chambers.

It is also an object of the invention to provide a technique for reducing problems arising from differences in the rate and extent of growth of tumor cells in the assay which can lead to 'overgrowth' which could lead to inaccurate results when using optical density measurements to quantitate cell growth.

The above objectives are achieved according to the inventive process to detect the presence of 'overgrowth' of the cell population. The process in comprised of the following steps:
    a) inoculating three culture chambers with three or more different concentrations of cells (number of cells/ml);
    b) growing the cell samples in the presence of growth-promoting medium for a given duration and then optically measuring the density of each of the control cell samples;
    c) establishing a graphical or mathematical relationship between the optical density measured with the two lowest concentrations of cells and the respective concentration of cells in the original samples;
    d) using that mathematical or graphical relationship to extrapolate to the third sample and to calculate the ratio between the extrapolated (expected) optical density for that sample, and the actual measured optical density; and,
    e) using this ratio as a basis for rejection of assays in which 'overgrowth' has occurred. According to the preferred embodiment, at least two of the three cell samples are control samples of lesser cell concentration than the third sample which has a cell concentration substantially the same as the cell concentration which is used in the test inoculum for the assay. Following the growth of the cells, a substantially linear relationship is defined between the measured optical density of the two lesser cell concentrations. This linear relationship is used to mathematically extrapolate to predict the 'expected' optical density value for the third control cell concentration. The ratio between the 'expected' value and the 'actual' measured value is calculated and if this ratio is unacceptably large then the assay is rejected.

Most preferably the cells are cancer cells and the process comprises inoculating four cell control samples in the ratios of 1:2:4:8 cells, ie. 250, 500, 1,000, and 2,000 cells/16 mm diameter well.

The assays may be chemosensitivity or radiosensitivity assays, or other assays in which the toxicity of an agent for normal or tumor cells is under examination.

Preferably an apparatus is provided for automatically detecting 'overgrowth' or 'overproliferation' of cells by comparing the extrapolated 'expected' optical density values to the 'actual' observed values. Such apparatus is preferably a suitably programmed computer connected to an apparatus for measuring optical density. The programmed computer would define the substantially linear relationship between the measured optical density values of at least two of the samples, which will include means for extrapolating to calculate the 'expected' optical density values at the higher cell inoculum concentration. These computing means may also include methods for calculating the deviation between the 'expected' and the 'actual' observed values, and may also include means for automatically rejecting the assay if the deviation is unacceptable.

It is further an object of the invention to provide a method and apparatus for quantitiating cell colonies which is objective, (rather than subjective- as is the case with prior clonogenic assays,) and readily suited to automation.

The above objectives are achieved according to the inventive process by fixing the cell colonies with an alcohol, formaldehyde, or other commonly used histological fixative; staining them with a dye (such as, but not exclusive to, crystal violet, or Giemsa, or May Grunwald Giemsa); illuminate the sample with light; and, measuring the optical density of the sample. According to the inventive process the colonies are tumor cells or normal cells and the process is comprised of:

a) inoculating culture chambers with 250 to 16,000 cells/16mm diameter well;

b) including in the assay a control well which is not inoculated with tumor cells;

c) growing the cell samples in the presence of growth-promoting medium for a given duration;

d) fixing the cells with a suitable histological fixative, such as, but not exclusive to, alcohol or formaldehyde, and subsequently staining the cell colonies with a dye, such as, but not exclusive to, crystal violet or May Grunwald Giemsa;

e) illuminating the stained cell colonies and optically measuring the density in each of the cell cultures.

In a sensitivity assay each of the assay chambers would be exposed to a different treatment modality, eg. a different dose of chemotherapeutic drug or a different dose of ionizing radiation, the cell colonies surviving the agent grow and their growth is quantitated by fixing, staining, and measuring the optical density.

It is further an object of the invention to provide a method for quantitating and subtracting 'background noise' contributed to the assay by non-growing cells or clumps of non-cellular tissue debris contributed by the original specimen. The method comprises adding an agent to an inoculum which kills the dividing cells, after which this 'sterile' inoculum is added to the culture chamber in the assay. In this manner, at the conclusion of the assay the contribution of the original specimen to the total optical density can be measured and subtracted. The agent is selected from the group of compounds consisting of: tritiated thymidine, colchicine, and mitomycin C, although other agents operating in the same fashion to accomplish substantially the same result may quite obviously be used.

Another object of the invention is to provide a method for mathematically adjusting and controling for the multiple variations in the assay to calculate assay results which will provide accurate, precise, and sensitive data describing the toxicity of the various agents under test on the particular cells being tested. This data is then used to establish the relative sensitivity or resistance of the cells being tested to the agent under test.

It is further an object of the invention to provide a method for analyzing assay results which readily lend itself to computerized data manipulation and evaluation.

These objects are accomplished in combination with the other assay steps (detailed above) throught the following additional steps consisting of:

a) treating a plurality of cell samples in the assay with the agent under test at different treatment doses or with different modalities of agent;

b) measuring the efficacy of each treatment with optical density measurements to quantitate cell growth (as detailed above);

c) calculating the validity of the assay through the use of cell proliferation controls for 'overgrowth' or 'undergrowth' (detailed above);

d) subtracting the non-specific 'background noise' from the assay results through the use of the background subtraction controls (detailed above);

e) plotting or mathematically expressing the data in the form of a 'survival curve' where the results are expressed to show the correlation between cell growth (as expressed by optical density measurements) and the treatment dose or modality;

f) determining mathematically the dose or modality at which 90% of the cells are killed by the agent (termed the 90% cytotoxicity or IC90 value); and

g) using the IC90 value to determine the relative efficacy of the particular treatment agent.

Most preferably the process of analysis is automated by establishing a continuous data line between the measurements taking place in step b) and the mathematical functions which can be computerized in steps c) through g). The treatment may take the form of treating the same number of tumor cells with different doses of a chemotherapeutic agent, or with different doses of radiation.

The method further includes storing at least one value from the assay, the IC90 value, either in a notebook or in a computer so that a comparison can be made between the relative treatment efficacy of an agent on cells derived from different patients, or between different agents on the same cells. Most preferrably a comparison will be made of the sensitivity of tumors from different patients to the same chemotherapeutic agent, or to the same dose of radiation, In this particular preferred embodiment a method is accorded for statistically ranking the relative efficacy of different cancer agents for killing or slowing the growth of a patient's tumor. In other embodiments the relative value of different existing (or newly developed) chemotherapeutic agents, drugs, or toxic agents can be statistically ranked, with respect to their ability to kill or slow the growth of cells including tumor cells or normal cells derived from any mammalian or animal source.

## DRAWINGS

Embodiments of the invention will now be described with reference to the drawings, in which:

Figure 1A is a plot of optical density (vertical axis) versus relative initial cell counts for a series of 'inoculum control samples' (see Example 2 below);

Figure 1B is a plot (Radiation Survival Curve) of optical density (vertical axis) (logarithmic scale) versus radiation dose in Gray (see Example 2 below);

☐ 16K inoculum

■ 8K inoculum

Figure 2A illustrates survival fraction of different cell types versus radiation dose in Gray (Gy) (see Example 3 below); Note that these survival curves do not show plateaus at the terminal portion of the curves.

Figure 2B illustrates survival fraction or cells versus different doses of various cytotoxic drugs (see Example 3 below); Note that these survival curves do not show plateaus at the terminal portion of the curves.

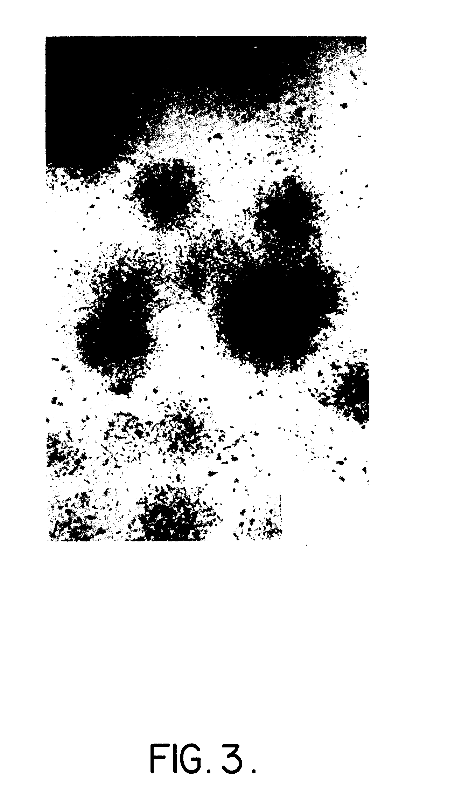

Figure 3 is a photograph of stained cell culture (see Example 3 below) showing typical colony formation at the end of the culture period. Thirteen colonies are apparent in this photograph. Ten of the colonies have sizes ranging from about 75 cells to about 250 cells. These ten colonies probably derived from single colony forming cells. The two large colonies contain in excess of 3000 cells and probably derived from aggregated colony forming cells. A colony assay would score these large colonies as deriving from a single colony forming cell whereas the inventive procedure would score these large colonies as deriving from multiple colony forming cells.

Figure 4 shows survival curves (% survival vs. drug [cisplatin] concentration) for tumor cells from three different individuals tested in vitro (see Example 5 below); and

Figure 5 is a graph of the numbers of individuals in a large group, whose tumor cells has IC90 values at various concentrations of a cytotoxic drug (cisplatin) (see Example 5 below) (Distribution of responses to Cisplatin). The median is at 0.3 ug/ml. Cells falling below 0.18 ug/ml are considered to be sensitive.

## DETAILED DESCRIPTION OF THE PREFERRED EMBODIMENTS

The inventive technique is preferably employed in conjunction with assays testing the toxicity of chemical or physical agents for cells, such as, but not exclusive to chemosensitivity or radiosensitivity testing of tumor cells.

The assay is performed by using a cell culturing technique in which cell growth is optimized through the use of: a) an extracellular matrix material coated onto the surface of the tissue culture chamber such as glass or plastic and including polystyrene plastic; and, b) a suitable liquid medium promoting the growth of the cells which contains 0.01% to 65% serum (either bovine, equine, porcine, human, or other) , and/or other growth and nutrient factors promoting cell growth such as, but not exclusive to, tissue culture medium containing insulin, insulin-like growth factor, epidermal growth factor, platelet derived growth factors acidic or basic fibroblast growth factor. The extracellular matrix material promotes attachment of cells requiring 'anchorage dependence' for growth and when used in combination with the liquid tissue culture medium cells growth is promoted so that cultures can be established from human humor biopsies including lung cancer, melanoma, sarcomas, breast cancer, and other primary or metastatic tumors.

Cell suspensions are obtained from tissue and organ samples by means which are standard to one skilled in the art, involving mechanical methods such as, but not exclusive to, mincing the tissue with a scalpel and scissors, and digesting it with enzymes including collagenase to liberate the cells from the connective tissues, and centrifuging the sample to remove aggregates of cells and non-digested tissue fragments.

The proper dispersion and uniform distribution of cells into the tissue culture assays is the key to achieving reproducible results. Local accumulation of cells at the edges of tissue culture chambers (ie. the wells in tissue culture plates, cr the edges of tissue culture flasks cr plates), leads to locally high concentrations of cells. The addition of a viscosity modifier according to the invention has the effect of decreasing the capillary forces at the edges, and producing uniform distribution of cells in the tissue culture chamber.

According to the preferred embodiment cf the invention methylcellulose is added to the liquid medium in which the cells are dispersed at a concentration of 0.6%. The range of methylcellulose is from 0.3% to 0.8%, preferably 0.5% to 0.7%, and most preferably 0.6%. Although methylcellulose is preferred for purpose to the invention, it is to be understood that the invention is not limited to any one particular viscosity modifier and extends to all modifiers.

The amount of viscosity modifier used is a function of the type of modifier used and is determined empirically through the addition of different amounts of modifier to a cell suspension containing 2,000-500,000 cells/ml. The cells may be selected from the group comprising: cells from primary human tumors or normal tissues, and cells from established human and animal cell lines. The resultant dispersed cell solution is added to coated tissue culture chambers and the cells are allowed to adhere the surface of the chamber during incubation at 37C with 5-7% $CO_2$ in air. After a suitable period of time (usually 6-24 hrs.) the dispersion medium is removed, the cells are gently washed with 0.01M phosphate buffered, 0.14M sodium chloride (PBS), and this is replaced with growth medium. After a suitable period of time (usually 5 to 13 days) the cell colonies which have grown are fixed and stained and visually examined for uniformity of distribution of the original cellular inoculum.

The invention also finds application in connection with methods for producing Controls for cellular 'overgrowth' in the assay. These Controls are most important to insure that toxic agents are tested optimally, eg. during the logarithmic-phase of cell growth, and to insure that the results of the assays can be properly interpreted. It is especially important to use these Control methods for defining the tolerances in assays where automated optical density measurements may be used to quantitate cell growth.

Briefly, the assay begins as described above by adding dispersed cells, in liquid medium containing an appropriate viscosity modifier, to the tissue culture chambers coated with a suitable extracellular matrix material. The cells are added in the amount of 2,000-500,000 cells/ml and in the preferred embodiment the tissue culture chamber is a 24 well tissue culture plate where 1ml of cell suspension is added to each well. The tissue culture plates are incubated for 6-24 hours at 37C with 5-7% $CO_2$ in air, to allow the cells to adhere to the surface of the wells, and at the conclusion of this time the wells are gently washed with PBS, and fresh tissue culture growth medium is then added.

For drug or toxic agent testing, the fresh medium contains the drugs or toxic agents to be tested at different doses or with different treatment modalities. For radiation sensitivity testing, the cells in wells are irradiated with different doses of radiation.

On the sixth day of all assays, the medium is exchanged for new fresh medium which does not contain drugs or toxic agents, the cultures are check for spot contamination or 'no growth' of the test inoculum, and the cultures which are growing are incubated for an additional 7 days at 37C in 5-7% $CO_2$. Contamination cultures are 'sterilize', so that they do not affect their neighbors, by the addition of a toxic agent, preferably 95% ethanol.

The assays are terminated by fixing the cells with ethanol (or a suitable histological fixative) and stained with crystal violet (or another suitable dye) and illuminated with light to determine the optical density of the individual stained culture wells.

For drug or toxic agent testing, 1 to 4 multi-well plates are inoculated permitting the testing of 3-9 drugs in duplicate at 2-6 different dosages over a relatively narrow range of concentrations, depending upon the yield of cells obtained from the original tissue or tumor sample. The doses of different drugs to be used in the assay are initially determined using the highest concentration as the concentration which is equal to the known bone marrow toxic dose of the drug. The doses may then be empirically adjusted downward and 'fine-tuned' by using the accumulated experience with the drug in the assay. Drug dose sets are prepared in 0.2ml aliquots at a 20 fold higher concentration than that which they will be used in the final assay. These concentrated 'stock' solutions are then stored frozen at -70C until use.

The controls in the drug or toxic agent sensitivity assay include:

a) the blank control: coated tissue culture wells (in the preferred embodiment usually at least two) which are not inoculated with cells (as control for the contribution of the coating surface to the final measured optical density of the culture, this control is referred to as the 'matrix' control);

b) the background control: a control which is another subject of the inventive process involves inoculating a coated culture well (or duplicate wells) with a specially prepared 'sterile' (dead) inoculum (as control for variation in the contribution of the inoculum to the final measured optical density of the cultures);

c) the inoculum control: another set of at least three controls, which is the subject of another inventive process, involving inoculating coated wells with cells prepared at three different cellular concentrations, with the inoculum preferably consisting of cell numbers in the ratios of 1:2:4:8, for example, but not exclusively, 250, 500, 1,000, and 2,000 cells/16 mm diameter well as a control for ascertaining cell 'overgrowth' (which may be referred to as the 'inoculum titration' control). A 100% reference point for the assay samples, can be determined from the foregoing set.

The inventive technique used to prepare the background control which is the subject of step b) above, comprises exposing the cell suspension to a agent which will kill the cells and render the inoculum 'sterile' of growth. The preferred agent according to the invention is an agent which kills off only the dividing (cancer or normal) cells and leaves behind the non-dividing (dead) debris so that their contribution to optical density can be measured. Such an agent is preferably, but not exclusively, selectived from the group comprising: tritiated thymidine, colchicine (Colcemide-tm), and mitomycin C. The tritiated thymidine is generally added in amounts of: 0.5-5 microcuries per ml, more preferably 1 to 5 microcuries per ml, and most preferably 5 microcuries per ml. Colchicine is generally used in amounts of 1 to 10 ug/ml, and most preferably 2ug/ml.

The controls for testing radiation sensitivity require approximately 2 multi-well plates. The assay is run at 6 dosages of radiation with duplicate wells being treated at each dose. The doses are 0.5-6 Gray (GY, a measure of radiation). The controls are the same as those detailed above, and the cultures are also 'fed' on day six with fresh medium, as detailed above. Cultures are terminated by fixing and staining in a manner identicle to that detailed above.

Upon completion of the assay according to the process of invention the optical densities of the wells is determined by illuminating the individual tissue culture walls, measuring the optical density of each culture using an optical image analyzer, such as, but not exclusively, a Nikon Magiscan automated image analyzer. Quite obviously, any alternative available means may be used as well for purposes of measuring the efficacy of the therapeutic agent, eg. cell colony counting, etc.

Next, the optical densities of the lowers two or three wells in the 'inoculum titration' control are plotted versus the initial cell inoculum concentration. Ideally this would result in a linear plot, and the data is preferably fitted as closely to a linear mathematical model as possible. However, at higher cell inoculum concentrations where 'overgrowth' may have occurred the optical density measurements show lower values than predicted by a linear extrapolation of the lower inoculum values, and in this case only the lower (linear) portion of the curve are to be considered.

The final calculated linear relationship between cell inoculum concentration and optical density is used to mathematically extrapolate to the 'expected' optical density measurement for the cell inoculum as the inoculum in the sensitivity assay, which is the control inoculum of step c), p. 14 above. The ratio between the 'expected' optical density and the 'actual' optical density of the 'positive control' is calculated. It this ratio is unacceptably high, it is used as the basis for excluding the assay from consideration.

Evaluation criteria for the acceptable ratio of 'expected' to 'actual' optical densities is a function of the 'kill rate basis' on which the assay is performed. Ideally an acceptable assay has occurred when:

ODe/ ODa is less than 100/Kr

where:

ODe = the extrapolated optical density value or 'expected' value;

ODa = the 'actual' measured value for the positive control; and,

Kr = the inhibitory value in the form of % survival in the assay. Preferably this is 10%, but another survival rate can be selected (50%).

Finally, in accord with the process of the invention, the corrected data is used plotted to obtain a 'survival curve', where 'survival', or its converse 'killing', (as measured by optical density measured in the treated cultures or by other obvious available methods such as colony counting) is plotted versus the dose or modality of toxic agent used as a treatment. In the preferred embodiment for drug sensitivity testing, this involves plotting in a semi-log plot the corrected optical densities for the test samples against the concentration of drug used for treatment in the assay (eg. 'survival' or 'kill' on the log ordinate with concentration on the linear abcissa). The results can be mathematically fitted to a curve using any of a variety of standard curve-fitting techniques, including, but not exclusive to, linear regression analysis of the data. Fitting the data to a curve can also be obviously accomplished automatically, eg. by a computer. The drug concentration, dosage, or modality at which 90% of the cells are killed (or 10% are surviving), (as indicated by the optical density which is 10% of the adjusted 'positive control' value), is defined as the IC90 value for the drug or toxic agent under test for that particular cell. Since a value of growth (i.e., survival) is selected (i.e., 10%), the value of the dose which produces such a growth is defined as an "inhibitory value." The inhibitory value, in this case the IC90 value, can be automatically calculated, eg. by a computer, and the 'survival curve' in whole or in part and IC90 value can be archived for future reference in computer data files or in notebooks. By relating accumulated experience to these data files the invention accords a method for statistically 'ranking' on a percentile scale the relative sensitivity or resistance of cells to a particular series of agents.

In the preferred embodiment for chemotherapy drug sensitivity, radiation sensitivity, or toxic agent testing, the response of a particular patients' tumor cells to a particular agent, as defined by the survival curve (in whole or in part) and IC90 value, is compared with the response of other archived results which are assembled in a percentile histogram where the percentage of test samples observed with a particular sensitivity or resistance to the test agent is plotted on the ordinate against the concentration of the agent on the abscissa. Thus the relative clinical potency of a test agent is ascertained.

In the preferred embodiment for drug sensitivity testing, the percentage of patient samples showing a given IC90 value is calculated at each concentration of drug and this is plotted on the ordinate against the concentration of drug, on the abscissa.

In the embodiment for radiation sensitivity testing, the percentage of patient samples showing a given IC90 value is calculated at each radiation dosage and this is plotted on the ordinate against the radiation dose in Gray (GY) on the abscissa. The position of particular test values on the histogram determine the relative 'responsiveness' or 'sensitivity' of the test sample to the particular agent. Sensitivity is suggested if the percentile from the histogram is low, eg. very few patients have tumors which respond to the drug (or radiation) at such a low concentration of drug (or radiation).

Alternatively, for radiation sensitivity testing a selected value for dose may be chosen, and the growth (i.e., survival or kill) at that selected dose (typically 2 Gray) is measured as the "inhibitory value". Such an inhibitory value can then be compared with inhibitory values for cells from other individuals.

At least in the situation where a selected value of dose is chosen, it may also be possible to use only one assay cell sample. This may be possible for either drug or radiation sensitivity testing. In such case growth values at that dose, can be compared between individuals.

The invention thus accords methods for accurately indicating the relative treatment efficacy of different drugs and physical agents which could be used for the treatment of a patients' cancer. Alternatively, the invention accords methods for accurately comparing the toxicity of different chemical or physical agents on normal cells derived from human and animal tissues. The following EXAMPLES illustrate various aspects of the invention.

EXAMPLE 1.

The following example is given to illustrate the use of a viscosity modifier in the inoculation of cell cultures.

Biopsy samples of tumors were obtained from cancer patients during routine diagnostic or therapeutic procedures.

The solid tumor biopsies were minced with scalpels to 1mm pieces. To prevent coagulation in fluid tumor samples heparin was added as an anticoagulant at a concentration of 10units/ml. The tumor cells in these samples were recovered by centrifugation. The tissues from either the solid or the fluid specimen were disaggregated by overnight incubation with constant stirring in tissue culture medium containing 0.075% collagenase type III (Cooper Biomedical, Malvern, PA.), 0.05% DNase (Sigma, St. Louis, MO.), and 10% fetal bovine serum.

After overnight enzymatic digestion, the resulting cell suspension was washed free of enzyme solution by centrifuging the digest and resuspending the cell pellet in 1 to 10ml, depending upon the size of the pellet, of phosphate-buffered saline (PBS). A small aliquot (0.05ml) is removed from the cell suspension, mixed with an equal volume of trypan blue dye (0.5%), and the viable tumor cells in the sample are counted using a microscope and hemocytometer counting chamber. This process is aided through the use of a phase gradient imaging system for visualizing viable cells. Briefly, the criteria for viability are: 1. the cells are not stained by trypan blue; 2. they have a relatively large size ($> 15$ microns); 3. they have a large nucleus to cytoplasmic ratio; 4. nucleoli are prominent in the nucleus; and, 5. on phase contrast examination they have nil to moderate granularity. The number of viable tumor cells counted at 400X magnification is used to calculate the cell concentration (cells/ml) and an aliquot containing the number of cells necessary for the assay (ie. approximately 200,000 cells for a 9 drug sensitivity assay) is withdrawn and diluted to the final desired concentration of cells (ie. 2,000 cells/ml) with 'attachment medium' which is alphaMEM containing 0.6% methylcellulose in addition to other supplements: namely, transferrin (10mg/ml), hydrocortisone (0.5mg/ml), HEPES buffer (2.7mg/ml), epidermal growth factor (5ng/ml; Collaborative Research, Lexington, MA), insulin (5mg/ml), antibiotics (penicillin plus streptomycin; 100units/ml) and 10% fetal bovine serum or swine serum (J.R. Scientific, Woodland, CA.). One milliliter of the final cell suspensions is used to inoculate 24 well tissue culture plates (Costar, Cambridge, MA.) precoated with cell adhesive matrix (Lifetrac, Irvine, CA.) or extracellular matrix material (IBT, Jerusalem, Israel). Use of the viscosity modifiers in the 'attachment medium' (0.6% methylcellulose) prevents aggregation of the cells while they are adhering to the surface of the wells, and also serves to maintain a uniform cell suspension which is not easily disturbed by vibration of disturbances occuring during handling. The cells slowly "rain" down through the viscous attachment medium over a period of approximately 2-6 hours, and after 2 to 12 hours of contact with the culture surface the cells begin to a firm adherence to the surface. The adherence process is usually most fully developed at 24 hours. After 24 hours the 'attachment medium' was carefully removed from the culture wells, the attached cells were gently washed with PBS 2-3 times, and finally complete tissue culture medium added to the wells of consisting of the drug or toxic agent to be tested dissolved or suspended in alphaMEM supplemented with transferrin, hydrocortisone, HEPES buffer, epidermal growth factor, insulin, antibiotics, and 10% fetal bovine serum (all at the concentrations indicated above).

The advantages of the viscosity modifier were seen by contrast with early studies, in which the 'attachment medium' consisted of tissue culture medium which did not contain a viscosity modifier. In these studies there was a rapid and market redistribution of the cells to the periphery of the culture wells before the cells had time to adhere to the surface. Environmental vibration in handling and in tissue culture incubators had a particularly prominent effect with the cells gradually being vibrated into the deeper medium at the edges of the culture wells. Such problems were particularly marked in incubators having forced air circulation of the $CO_2$ atmosphere using electrical blowers. The error introduced into these assays by peripheral attachment of the cells was highly variable from well to well, plate to plate, and at different positions in the tissue chamber incubator. The variable contribution of this error in the final results was difficult to quantitate and therefore difficult to remove from the final calculations.

EXAMPLE 2.

The following example is given to illustrate the use of the 'inoculum control samples' used in the assays to control for 'overgrowth' of the cells in the assays.

The example illustrates the use and validity of the invention, in conjunction with an assay to test the sensitivity of human tumor cells to ionizing radiation. The protocol for this example is taken from Baker et.al., Drug and Radiation Sensitivity Measurements of Successful Primary Cultures of Human Tumor Cells, Cancer Research, Vol. 46, pp. 1263-1274, 1986 the disclosure of which is hereby incorporated by reference thereto.

The cellular inoculum was prepared from human tumor tissues, as described above, and the cells were suspended in 'attachment medium', also described above, at a concentration of 16,000 cells/ml, 8,000 cells/ml, 4,000 cells/ml, 2,000 cells/ml, and 0 cells/ml. One milliliter of the cells is then added to each well of a 24 well microtiter plate precoated with extracellular matrix material, as detailed above. The cells were allowed to adhere to the surface of the coated wells, as described above, then washed, placed in fresh medium, and a series of test wells at 16,000 cells/mil and another series at 8,000 cells/mil, were each exposed to a different graded dose of ionizing radiation from 0.5 to 6 Gray.

The cultures are incubated for an additional 5 days at which time the medium is exchanged. The cultures are incubated for an additional 6 days, at which time they are fixed, stained with crystal violet. The cell growth is quantitated by measuring the optical density of each well.

The apparatus used to measure optical density is any appropriate apparatus, but is most preferrably a Nikon Magiscan Image Analyzer with the computer appropriately modified to read the optical density of individual wells in multi-well plates.

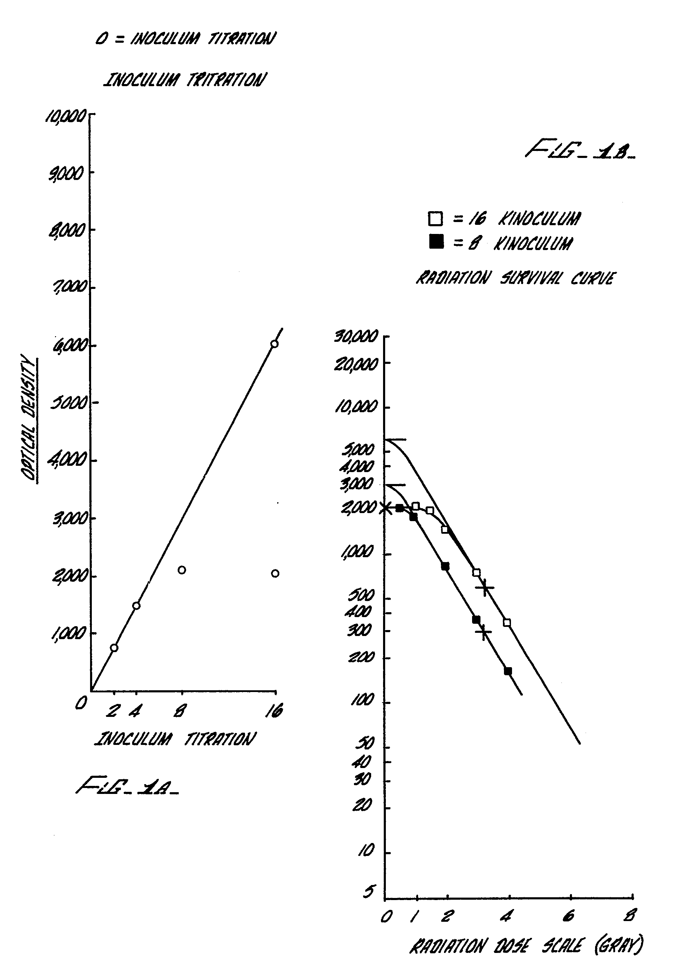

The optical density measurement of this inoculum titration are shown in Figure 1A. In this example, the optical density increased linearly with increasing numbers of cells in the inoculum up to 4,000 cells per well. The optical density continued to rise at 8,000 cells/well, but at 16,000 cells/well the loss of linearity becomes evident. In this example, the deviation from linearity occurred between an inoculum of 4,000 and 8,000 cells per well and the extrapolated value of the 'expected' optical density at 16,000 (the inoculum number of cells in the test wells) is determined by estrapolating the line drawn between the 0, 2000, 4,000 and 8,000 cell optical density measurements. The extrapolated 'expected' value of the 16,000 inoculum density in this example is 6,000, and the ratio of the 'expected' to the 'actual' value for the 16,000 inoculum is: 6000/2050 = 2.93.

To determine the level at which value of the ratio between 'expected' and 'actual' becomes unacceptable, the ID90 values (the radiation dose where 90% of the cells are killed) were determined for both the 8,000 and 16,000 cell/well inoculation concentrations. This was done by exposing duplicate wells inoculated with either 8,000 or 16,000 cells/well to ionizing radiation in the doses of 0.5-5 GY. The results presented in Figure 1B, shown that the ID90 values determined for each of the inoculum concentrations is identical, so in this case the ratio of 'expected'/'actual' = 2.93 (calculated above) is an acceptable ratio for the radiation sensitivity assay. In addition, these results validate the use of the extrapolated value for the 'expected' value in cultures where the 'positive controls' (above) exhibit 'overgrowth', eg. it may be possible using this method to interpret the data even if the 'positive controls' are 'overgrown'. Thus, in the example the 16,000 cell/well positive control is 'overgrown' but the extrapolated value for the 'expected' value of the 16,000 cell/well positive control was 6,000 (Figure 1A). The optical density where only 10% of the cells are still surviving (eg. the ID90 value) would be 10% of 6,000 or 600. The point on the survival curve where a value of 600 was seen is 3.3 Gray, and this value agrees well with the observed IC90 value obtained from the non-overgrown wells which were inoculated with 8,000 cells (Figure 1B) rather than 16,000. Thus the technique of comparing the ratio of

$OD_e/OD_a$ to 100/Kr as described, is validated.

EXAMPLE 3.

The following experiment is shown to illustrate that drug and radiation survival curves of primary human tumor cells generated from optical density measurement of growth avoids the counting (multiplicity) problems explained above.

Tumor Cell Preparation

Biopsies of tumors were obtained from cancer patients during routine diagnostic or therapeutic procedures. The biopsies were transported to the laboratory.

The solid tumor biopsies were minced with scapels to 1 mm pieces. Fluid specimens were anticoagulated with 10 Units/ml of Heparin and the cells recovered by contrifugation. The tissue from either the solid or fluid specimens were disaggregated by overnight incubation with constant stirring with the enzyme cocktail consisting of collagenase (0.075%) and DNase (0.005%) in culture medium supplemented with 10% fetal bovine serum.

After the overnight enzyme digestion, the resulting cell suspension is washed free of the enzyme solution by centrifuging the digest and resuspending the cell pellet in 1 to 10 ml of phosphate-buffered saline depending on the size of the pellet. A small aliquot (0.05 ml) is removed from the cell suspension, mixed with an equal amount of trypan blue (0.5%), and a tumor cell count is performed.

The tumor cell count is performed using a hemocytometer and a microscope fitted with a phase gradient inaging system so that living cells can be visualized. The number of viable tumor cells are counted at 400X magnification according to the following criteria: 1. unstained by the trypan blue dye; 2. large size (> 15 microns); 3. large nucleous to cytoplasmic ratio; 4. prominant nucleoli; and 5. nil to moderate granularity. The number of cells in 1/9th the marked area of the hemocytometer meeting the above criteria are counted.

The cell concentration is calculated and an aliquot containing the number of cells required for the assay (200,000 cells for a 9 drug assay using alphaMEM culture medium) is withdrawn.

The aliquot of cells required by the assay, is diluted with attachment medium to a final cell concentration of 2,000 cells/ml. Attachment medium is comprised of alphaMEM culture medium which contains 0.6% methylcellulose in addition to the other supplements: transferrin (10 ug/ml), hydrocortisone (0.5 ug/ml), HEPES buffer (5 mg/ml), epidermal growth factor (5 ng/ml), insulin (5 ug/ml), antibiotics, and 10% fetal bovine serum. Multiwell plates (Costar, Cambridge, MA, U.S.A.) precoated with Cell-Adhesive Matrix (CAM), or ECM (IBT, Jerusalem, Israel), are inoculated with 1 ml per well of the cells suspended in attachment medium.

Culturing

Multiwell plates precoated with CAM are inoculated with the cells as follows. Control cultures consising of: 1. inoculum control samples consisting of 2,000, 1,000, 500, 250, and 0 cells per wall are used to establish the 100% survival level; and 2. an inoculum of 2,000 cells per well incubated with a high dose of tritiated thymidine to establish the background level are set up in the first two columns of four wells. The remainder of the plate is inoculated with 2,000 cells per well for drug testing purposes.

After inoculation, the cultures are incubated under standard conditions for 24 hours to allow the inoculated cells to firmly adhere to the culture surface. After the 24 hour adherence period, the supernatant medium is removed, the adherent cells washed with phosphate-bufferred saline, and fresh supplemented alphaMEM culture medium is added. If the testing modality is radiation, wells are irradiated with graded doses of ionizing radiation over the range from 0.5 to 6 Gray using shields to confine the irradiation to individual wells. If the testing modality is chemotherapy drugs, culture medium containing drugs is added.

Four doses of drug are used which span a relatively narrow range, generally described by scale values 1:2:4:6 or 8 with reference concentration being the concentration of the unit scale value. Up to nine standard chemotherapy drugs are tested. One drug is tested in each column of four wells with the four doses applied to the four wells in the column with the lowest dose (scale value 1) in the top well to the highest dose (scale value 6 or 8) in the bottom well.

After the drugs have been added, or radiation applied, the cultures are incubated for 5 days after which the medium is exchanged for fresh culture medium thereby limiting the exposure to drug to five days. The cultures are then incubated for an additional 7 days after which the plates are fixed with ethanol (70%) and stained with crystal violet (0.5%).

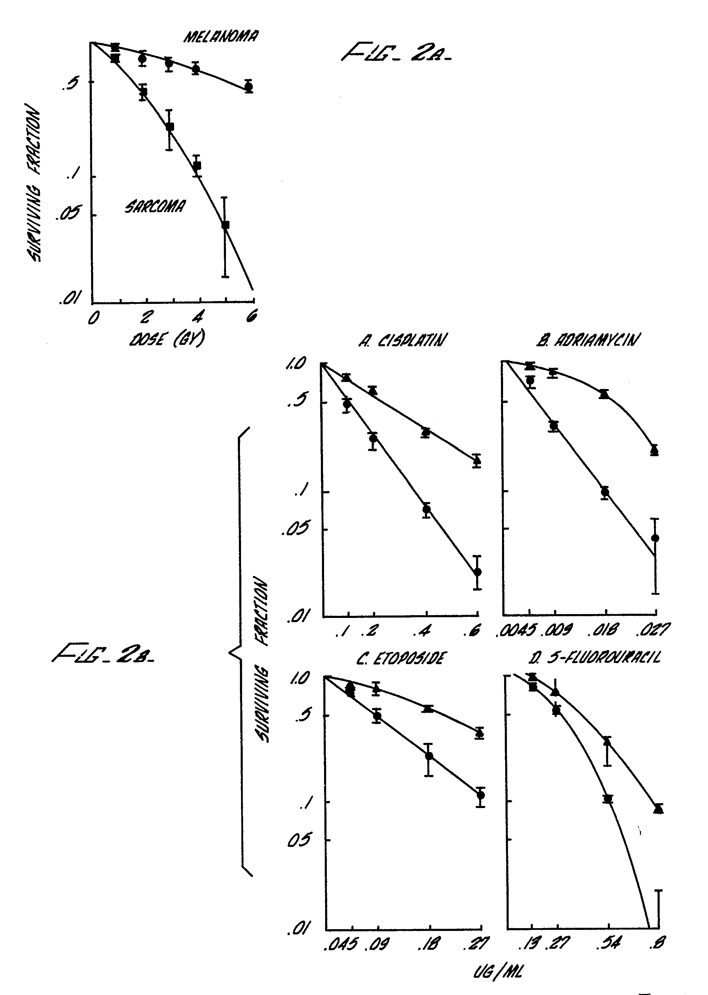

According to the inventive procedure, the cell straining density or optical density of each well integrated over the entire surface is measured by computerized image analysis which produces a number which is proportional to the cell staining density for each well. The surviving fraction for each drug tested is calculated using the 100% survival value and the optical density measurements for each drug dose tested. Survival curves are constructed using a quadratic least squares curve fitting computer program which returns the best fit line as illustrated in Figure 2A for radiation and in Figure 2B for drugs.

The survival curves shown in Figures 2A and 2B are typical. Of note is the absence of plateaus at the high dosage levels. Plateaus, particularly in radiation survival curves are caused by multiplicity (i.e., cell aggregation) and the absence of plateaus in the inventive optical density method of scoring suggests that this scoring method avoids the problems of multiplicity as follows. Generally a colony arises from one colony forming cell and the colony grows to a certain size and contributes a certain amount of cell staining density. However, as tumor cells often aggregate, a colony may have originated from an aggregate of two or more colony forming cells. In this case a colony arising from an aggregate of colony forming cells will be larger and contribute proportionally more to the cell staining density.

Figure 3 illustrates a culture that shows colony size heterogeneity consistent with this concept. The Figure is a photograph of a stained culture showing typical colony formation at the end of the culture period. Thirteen colonies are apparent in this photograph. Ten of the colonies have sizes ranging from 75 cells to about 250 cells. These ten colonies probably derived from single colony forming cells. The two large colonies contain in excess of 3000 cells and probably derived from aggregated colony forming cells. A colony assay would score these large colonies as deriving from a single colony forming cell whereas the inventive procedure would score these large colonies as deriving from multiple colony forming cells. Thus the cell staining density is proportional to the number of colony forming cells and multiplicity, which is a problem in colony assays, is compensated for in the inventive optical density measurement method for measuring growth.

EXAMPLE 4.
The following example is shown to illustrate the generation of the background value and its use in the construction of survival curves.

Cell Preparation
See Example 3.

Culturing
Multiwell plates precoated with CAM or ECM are inoculated with the cells as follows. Inoculum control samples consisting of an inoculum titration consisting of 2,000, 1,000 500, 250, and 0 cells per well are used to establish the 100% survival level.

According to the inventive procedure, a second set of control cultures are inoculated with 2,000 cells per well for the purpose of establishing the background. The remainder of the plate is inoculated with 2,000 cells per well for drug testing purposes.

After inoculation, the cultures are incubated under standard conditions for 24 hours to allow the inoculated cells to firmly adhere to the culture surface. After the 24 hour adherence period, the supernatant medium is removed, the adherent cells washed with phosphate-bufferred saline, and fresh supplemented alphaMEM culture medium is added.

According to the inventive procedure, culture medium containing tritiated thymidine at 5 uC/ml is added to the second set of control cultures also after the 24 hour adherence period.

Depending on whether drug or radiation sensitivity is being performed, either drugs or radiation is applied to the remaining wells also after the 24 hour adherence period.

The cultures are incubated for 5 days after which the medium is exchanged for fresh culture medium thereby limiting the exposure to drug to five days. The cultures are then incubated for an additional 7 days after which the plates are fixed with ethanol (70%) and stained with crystal violet (0.5%).

The cell staining density or optical density of each well integrated over the entire surface is measured by computerized image analysis in the manner previously described which produces a number which is proportional to the cell staining density for each well.

The background value is subtracted from the control value to obtain the 100% survival value and from all remaining optical density values prior to calculating the surviving fraction for each dose of either modality (drugs or radiation) being tested.

The surviving fraction for each drug tested is calculated using the 100% survival value and the optical density measurements for each drug dose tested. Survival curves are constructed using a quadratic least squares curve fitting computer program which returns the best fit line.

The end point is the concentration of drug that killed or inhibited 90% of the growing population of cells (IC90) and this is calculated by determining the intersection of individual survival curves and the point defined as 10% suvival which is the same as 90% kill.

EXAMPLE 5

The following experiment is shown to illustrate the generation of the multiple point survival curves and to illustrate how the curves reflect differences in sensitivities in patient of varying responsiviness to therapy.

Cell Preparation

See Example 3.

Culturing

Multiwell plates precoated with CAM are inoculated with the cells as follows. Control cultures consisting of: 1. inoculum control samples consisting of 2,000, 1,000, 500, 250, and 0 cells per well are used to establish the 100% survival level; and 2. an inoculum of 2,000 cells per well incubated with a high dose of tritiated thymidine to establish the background level are set up in the firt two columns of four wells. The remainder of the plate is inoculated with 2,000 cells per well for drug testing purposes.

After inoculation, the cultures are incubated under standard conditions for 24 hours to allow the inoculated cells to firmly adhere to the culture surface. After the 24 hour adherence period, the supernatant medium is removed, the adherent

According to the inventive procedure, four doses of drug are used which span a relatively narrow range, generally described by scale values 1:2:4:6, with the reference concentration being the unit scale value. This scale range is used for modalities showing a generally linear survival curve. For modalities which show shouldered survival curves the scale range may be 3:4:5:6. Up to nine standard chemotherapy drugs are tested. In the preferred embodiment of the invention, one drug is tested in each column of four wells with the four doses applied to the four wells in the column with the lowest dose (scale value 1) in the top well to the highest dose (scale value 6) in the bottom well.

After the drugs have been added, or radiation applied, the cultures are incubated for 5 days after which the medium is exchanged for fresh culture medium thereby limiting the exposure to drug to five days. The cultures are then incubated for an additional 7 days after which the plates are fixed with ethanol (70%) and stained with crystal violet (0.5%).

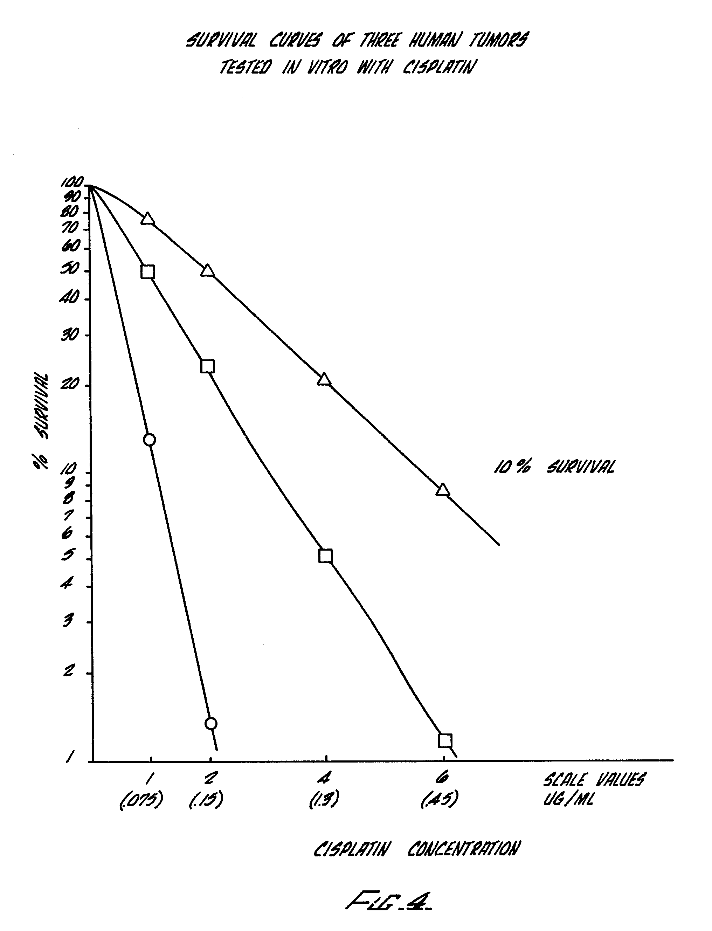

The cell staining density of optical density of each well integrated over the entire surface is measured by computerized image analysis which produces a number which is proportional to the cell staining density for each well. The surviving fraction for each drug tested is calculated using the 100% survival value and the optical density measurements for each drug dose tested. Survival curves are constructed using a quadratic least squares curve fitting computer program which returns the best fit line as illustrated in Figure 4.

The endpoint is the concentration of drug that killed or inhibited 90% of the growing population of cells (IC90) and this is calculated by determining the intersection of individual survival curves and the point defined as 10% survival which is the same as 90% kill. These doses can be seen in Figure 1 as where the survival curve intersects the horizontal 10% survival line. The IC90 scale values are converted to actual drug concentrations used in the assay by multiplying the IC90 scale value by the reference concentration which in the case of cisplatin is 0.075 ug/ml. The assay does not provide any data however as to the actual dosage to be administered.

The examples shown in this Figure 4 are typical survival curves for cisplatin for three patients of different sensitivity. The curve on the left has the smallest IC90 and is the most sensitive of the three. The curve on the right has the largest IC90 and is the least sensitive of the three and the curve in the middle is of intermediate sensitivity.

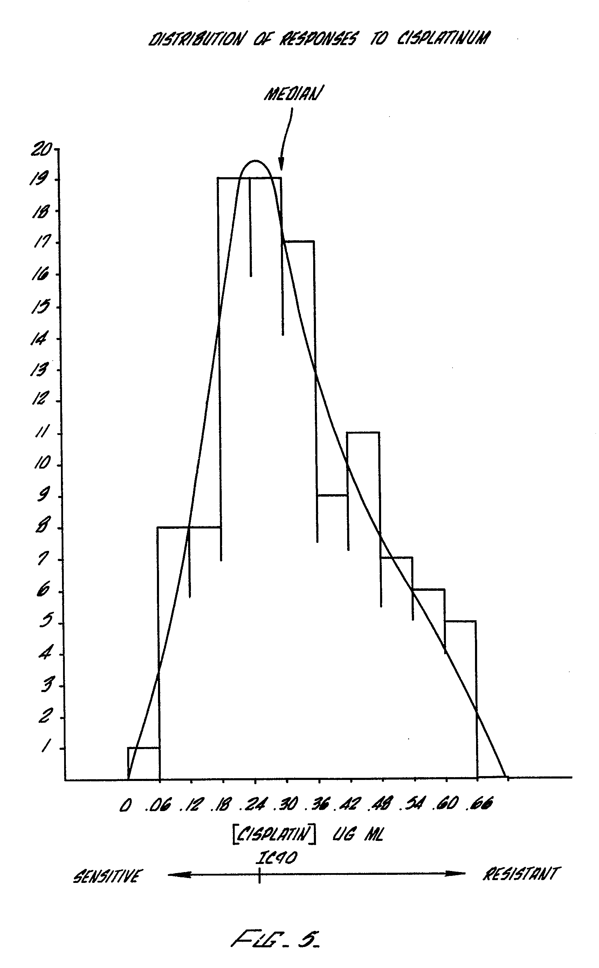

This type of analysis for IC90 values has been repeated for most common chemotherapeutic agents on over a hundred different primary human tumors of various histologies. The accumulated IC90 values show a normal distribution about a mean as illustrated in Figure 5 for cisplatin.

Sensitivity of a particular patient's tumor to cisplatin, for example, is determined by comparing the particular IC90 value to the distribution of IC90 values shown in Figure 3. In this instance the patient with the smallest IC90 would be considered to be sensitive to the drug since the particular IC90 is near the sensitive end of the distribution.

Summary statement.

As a result of the application of all the described improvements in the assaying of primary human tumor cells for drug IC90 vlaues, the coefficient of variation (mean divided by the standard deviation) was 3.95% determined from 5 assays on the same preparation of primary human tumor cells. It is well known that the agar suspension assay sytem has very poor reproducibility, which is probably in excess of 20% and it is considered that the execellent reproducibility of the inventive assay system is due to the combined application of inventions discussed in this application.

It is further understood by those skilled in the art that the foregoing description is that of preferred embodiments of the invention, and that various changes and modifications may be made in the invention without departing from the spirit and scope thereof.

## Claims

1. A process for determining the relative clinical potency of a test agent on cells from an individual, comprising treating a plurality of assay cell samples from the individual with different doses of the agent and measuring the growth of the cell samples, calculating an inhibitory value as the value of one of the dose and growth corresponding to a preselected value of the other one, then comparing the inhibitory value with inhibitory values for cells from a plurality of other individuals to ascertain the relative clinical potency of the test agent.

2. A process according to claim 1 wherein growth of the cell samples are measured by culturing the samples after exposure to the agent, then measuring their optical density so as to obtain a measure of the growth of cells in each sample which survived the treatment.

3. A process according to claim 2, additionally comprising treating a background control sample with a toxic agent so as to kill only dividing cells, and culturing it in the same manner as the assay samples, then measuring the optical density and subtracting the resulting value from the measured optical densities of the assay cell samples to obtain corrected optical densities for them.

4. A process according to claim 2 or claim 3 additionally comprising culturing a plurality of inoculum control samples in the same manner as the assay samples, each having a different initial cell count, then measuring the optical densities of the inoculum control samples and calculating the expected optical density of a particular inoculum control sample which would have the same initial cell count used in the assays based on the best linear relationship of measured optical density versus initial cell count for the inoculum control samples, and rejecting the assay results if the ratio of the calculated to measured optical density of the particular inoculum control sample exceeds a value which would indicate cell overgrowth in the cultured assay cell samples.

5. A process according to any preceding claim wherein the cell samples are placed in respective chambers, the method additionally comprising incorporating a viscosifier into each cell sample so as to inhibit cell aggregation and redistribution therein.

6. A process according to any preceding claim wherein the cells are tumor cells.

7. A process according to any preceding claim wherein the test agent is selected from chemotherapeutic drugs and radiation.

8. A process according to any one of claims 4 to 7 wherein the test agent is a cytotoxic drug and the cell count of the particular inoculum control sample is the highest of those control samples, and wherein the optical density measurements from the assay cell samples are rejected if the calculated to measured optical density ratio of the particular inoculum control sample exceeds the inverse of a selected survival ratio of the cells following exposure to a test agent.

0259183

O = INOCULUM TITRATION

INOCULUM TRITRATION

OPTICAL DENSITY

INOCULUM TITRATION

FIG_1A_

FIG_1B_

☐ = 16 KINOCULUM
■ = 8 KINOCULUM

RADIATION SURVIVAL CURVE

RADIATION DOSE SCALE (GRAY)

0259183

FIG. 2A.

FIG. 2B.

A. CISPLATIN
B. ADRIAMYCIN
C. ETOPOSIDE
D. 5-FLUOROURACIL

MELANOMA
SARCOMA

SURVIVING FRACTION

DOSE (GY)

UG/ML

0259183

FIG. 3.

0259183

SURVIVAL CURVES OF THREE HUMAN TUMORS
TESTED IN VITRO WITH CISPLATIN

FIG.4.

0259183

DISTRIBUTION OF RESPONSES TO CISPLATINUM

FIG. 5.